# EUROPÄISCHE PATENTSCHRIFT

(11) **EP 2 477 666 B1**
(45) Veröffentlichungstag und Bekanntmachung des Hinweises auf die Patenterteilung: **17.04.2013**
(21) Anmeldenummer: 11707566.3
(22) Anmeldetag: 25.01.2011
(51) Int. Cl.: A61L 27/24, A61L 27/38

(54) **VERNÄHBARES GEWEBETRANSPLANTATKONSTRUKT ZUR REKONSTRUKTION EINES MENSCHLICHEN ODER TIERISCHEN ORGANS**
TISSUE GRAFT STRUCTURE THAT CAN BE SUTURED FOR RECONSTRUCTING A HUMAN OR ANIMAL ORGAN
CONSTRUCTION DE GREFFE DE TISSU APTE À ÊTRE COUSUE POUR RECONSTITUER UN ORGANE HUMAIN OU ANIMAL

(30) Priorität: 27.01.2010 DE 102010001271
(43) Veröffentlichungstag der Anmeldung: 25.07.2012
(73) Patentinhaber: UroTiss GmbH, 01069 Dresden (DE)
(72) Erfinder: RAM-LIEBIG, Gouya, 01309 Dresden (DE)
(74) Vertreter: Carlsohn, Alexander
(86) Internationale Anmeldenummer: PCT/DE2011/075010
(87) Internationale Veröffentlichungsnummer: WO 2011/091796

(56) Entgegenhaltungen:
- EP-A1- 1 454 600
- DE-B3-102004 037 184
- US-A1- 2004 234 577

## Beschreibung

Die Erfindung betrifft ein vernäbares Gewebetransplantatkonstrukt zur Rekonstruktion eines menschlichen oder tierischen Organs, ein Verfahren zur Herstellung eines derartigen Gewebetransplantatkonstrukts sowie eine Verwendung des Gewebetransplantatkonstruktes. Die Erfindung betrifft insbesondere ein Gewebetransplantatkonstrukt zur Rekonstruktion eines Harnorgans, insbesondere der Harnblase, des Harnleiters oder der Harnröhre, des Ösophagus, der Augenoberfläche oder oraler Gewebedefekte.

Ein technisches Grundproblem, das bei der Implantation von Gewebetransplantatkonstrukten, die auf zellulärem Gewebe basieren, verhindert werden muss, ist die Ablösung des Gewebetransplantatkonstruktes aufgrund der normalen Peristaltik der Organe, beispielsweise der Harnblase, des Harnleiters oder der Speiseröhre. Bei der Rekonstruktion des Harnleiters und der Harnröhre besteht ein weiteres Problem darin, dass der Harnfluss eine angemessene und feste Fixierung des Transplantats am Wundbett erfordert. Dies gilt ebenso für die Rekonstruktionen im oralen Bereich oder im Bereich der Speiseröhre im Hinblick auf die Aufnahme von Speisen und Getränken. Bei der Rekonstruktion der Augenoberfläche ist eine solche angemessene und feste Fixierung des Transplantats aufgrund der Augenbewegung notwendig.

Ein derartige solche angemessene und feste Fixierung des Transplantats kann nur mit einer geeigneten Nähtechnik erreicht werden, die sicherstellt, dass mit dem Transplantat ein hinreichendes funktionelles Ergebnis erzielt wird und die ferner die Risken einer Blutung und einer Infektion verhindert. Zur Fixierung von Transplantaten muss die verwendete Nadel durch das Konstrukt und das angrenzende Gewebe geführt werden, um die Verbindungsnaht mittels des von der Nadel geführten Fadens herzustellen. Der Faden muss ausreichend fest angezogen werden, um eine angemessen Anpassung des angrenzenden Gewebes an den Rand des Transplantates zu erreichen. Bei einem vergrößerten Wundödem muss die auf den Faden angewendete Zugspannung erhöht werden. Überdies sind zur Verringerung der Wundkontraktion vielfach größer dimensionierte Nadeln und größere und tiefere Stiche erforderlich.

Aufgrund der vorstehenden Umstände ist eine Stabilität des Transplantates erforderlich, die so hoch ist, dass größer dimensionierte Nadeln und größere und tiefere Stiche tatsächlich ausgeführt werden können, ohne die Funktion oder den Bestand des Transplantats zu gefährden. Sobald eine ausreichende Anzahl von Fäden zwischen das Transplantat und das angrenzende Gewebe eingebracht ist, werden normalerweise Knoten mit einer Spannung gebunden, die ausreichend ist, um die Annäherung der Ränder zu ermöglichen und so ein Auseinanderweichen der Wundränder und eine Narbenbildung zu verhindern.

Aufgrund der hohen Zugfestigkeit des Transplantats, die aus den oben genannten Gründen erforderlich ist, müssen die Gewebetransplantatkonstrukte eine hohe mechanische Stabilität aufweisen. Bisher wurden verschiedene Materialien genutzt, die als Gerüst für die Gewebetransplantatkonstrukte auf Basis von zellulärem Gewebe dienten. Allen diesen Materialien mangelt es jedoch an der erforderlichen mechanischen Stabilität und der Vernähbarkeit. In den meisten Fällen können nur Knoten ohne Zuganpassung eingesetzt werden.

Es gibt einige Membranen wie Dünndarm-Submukosa, von denen berichtet worden ist, dass sie eine hohe mechanische Festigkeit aufweisen. In der lyophilisierten Form ändert sich jedoch die dreidimensiononale Kollagenstruktur derartiger Membranen. Aus diesem Grunde ist die Kultivierung von Zellen auf langen Segmenten dieser Membranen unter Ausbildung von Mehrfachschichten nicht möglich (Wei R et al., Grafts of Porcine Small Intestinal Submucosa with Cultured Autologous Oral Mucosal Epithelial Cells for Esophageal Repair in a Canine Model.,... Experimental biology and medicine. 2009:234. 453-461; Lindberg K et al., Porcine small intestinal submucosa (SIS): a bioscaffold supporting in vitro primary human epidermal cell differentiation and synthesis of basement membrane proteins. Bums. 2003:254-266).

Bekannt sind ferner Gewebetransplantatkonstruke, die autologe orale Mucosazellen umfassen, die auf einer equinen (TissuFoil) oder bovinen (Matriderm) Kollagen/Fibronektin-Matrix kultiviert wurden. Die Matrizes werden im Anschluss an die Geweberegeneration vollständig resorbiert und entsprechen komplexen Oberflächen. TissuFoil und Matriderm sind zertifizierte medizinische Erzeugnisse der Firma Baxter und Suwelack und sind für die Verwendung beim Menschen zugelassen. Sie ermöglichen eine sehr gute Zelladhäsion und -proliferation of ihren Oberflächen.

EP 1 002 031 beschreibt eine vernähbare Membran, die die Adhäsion der Membran an Prothesen oder anderen Kompensationsteilen verhindern soll. Die Membran umfasst einen erste Schicht aus einem Kollagenfaservlies und eine Schwammschicht aus Kollagen. Das Kollagen beider Schichten wird aus einer gemeinsamen Quelle gewonnen. Beispielsweise kann das für beide Schichten verwendete Kollagen aus Rindern, Schweinen, Geflügel, Fischen, Kaninchen, Schafen, Urin und Menschen gewonnen werden.

Aus US 2007/0161109 A1 ist eine azelluläre Membran bekannt, die das Gewebewachstum unterstützen soll. Die Membran umfasst eine erste Schicht und eine zweite Schicht, die jeweils Kollagenfasern aufweisen. Die Kollagenfasern werden aus einer natürlichen, nicht näher beschriebenen Quelle gewonnen. Eine mehrschichtige Membran aus Kollagen ein- und derselben Quelle ist ferner in US 7,393,437 und in US 2004/0234577 offenbart.

Die mechanische Stabilität bekannter Matrizes ist bei der Einwirkung von Zugkräften jedoch für eine wirksame Fixierung von Gewebetransplantatkonstrukten unzureichend. Es besteht Bedarf an einem Gewebetransplantatkonstrukt, das eine höhere Zugfestigkeit und verbesserte Vernähbarkeit aufweist. Ferner ist es wünschenswert, ein Gewebetransplantatkonstrukt bereitzustellen, das eine hohe Biokompatibilität aufweist und den medizinrechtlichen Anforderungen an deren Verwendung beim Menschen genügt.

Aufgabe der Erfindung ist es, die Nachteile nach dem Stand der Technik zu beseitigen. Es soll insbesondere ein Gewebetransplantatkonstrukt angegeben werden, dass verbesserte mechanische Eigenschaften aufweist und gleichzeitig für eine Kultivierung mit Zellen geeignet ist. Ferner soll ein Verfahren zur Herstellung eines solchen Gewebetransplantatkonstruktes sowie Verwendungen des Gewebetransplantatkonstruktes angegeben werden.

Diese Aufgabe wird durch die Merkmale der Ansprüche 1, 6 und 9 gelöst. Zweckmäßige Ausgestaltungen der Erfindungen ergeben sich aus den Merkmalen der Ansprüche 2 bis 5, 7 bis 8 sowie 10 bis 12.

Nach Maßgabe der Erfindung ist ein Gewebetransplantatkonstrukt zur Rekonstruktion eines menschlichen oder tierischen Organs vorgesehen, dass
(a) einen photochemisch vernetzten Membranverbund, umfassend zumindest eine erste biologisch kompatible, kollagenhaltige Membran und zumindest eine zweite biologisch kompatible, kollagenhaltige Membran, wobei die erste Membran und die zweite Membran an ihren Flachseiten aneinander angrenzen und wobei die erste Membran equinen oder bovinen Ursprungs ist und die zweite Membran tierischen oder humanen Ursprungs ist und die zweite Membran anderen Ursprungs als die erste Membran ist; und
(b) ein oder mehrere Schichten von autologen oralen Mukosagewebezellen auf einer oder beiden äußeren Flachseiten des Membranverbundes
umfasst.

Der Membranverbund kann eine oder mehrere erste Membranen umfassen. Ebenso kann der Membranverbund eine oder mehrere zweite Membranen. In einer bevorzugten Ausführungsform umfasst der Membranverbund zwei erste Membranen und eine zweite Membran, die zwischen den beiden ersten Membranen angeordnet ist. Auf diese Weise wird ein dreilagiger Membranverbund erhalten, der einen sandwish-artigen Aufbau hat. Dabei ist die Oberseite der zweiten Membran von einer ersten Membran bedeckt, wobei sich deren Unterseite und die Oberseite der zweiten Membran gegenüberliegen. Die Unterseite der zweiten Membran ist von der anderen ersten Membran bedeckt, wobei sich deren Oberseite und die Unterseite der zweiten Membran gegenüberliegen.

Es ist wesentlich, dass die erste Membran und die zweite Membran unterschiedlichen, d. h. heterologen Ursprungs sind. Unter dem Begriff "Ursprung" wird hier verstanden, dass die erste Membran und die zweiten Membran nicht von derselben taxonomischen Art stammen. Eine Membran equinen Ursprungs stammt vom Pferd. Eine Membran bovinen Ursprungs stammt vom Rind. Eine Membran porzinen Ursprungs stammt vom Schwein.

Das erfindungsgemäße Gewebetransplantatkonstrukt bietet verbesserte mechanische Eigenschaften, insbesondere eine hervorragende Vernähbarkeit und mechanische Stabilität, aufgrund des Einsatzes eines Membranverbundes, auf den einerseits die guten mechanischen Eigenschaften zurückzuführen sind und der andererseits Oberflächen aufweist, die eine gute Adhäsion von Zellen und deren rasche Proliferation unter Ausbildung von dichten Schichten auf den äußeren Oberflächen des Membranverbundes ermöglichen. Die ersten Membranen ermöglichen insbesondere ein gutes Zellwachstum in vitro. Ein gutes Zellwachstum liegt beispielsweise dann vor, wenn eine oder mehrere Schichten von Zellen auf der äußeren Oberfläche der ersten Membran wachsen, wobei die Mernbranoberfläche > 5cm², die Vitalität der Zellen für einen Zeitraum größer 48 h mindestens 90 % betragen sollte.

Die guten mechanischen Eigenschaften sind auf die Eigenschaften der zweiten Membran zurückzuführen, während die guten Eigenschaften in Bezug auf die Zelladhäsion und -proliferation auf die Eigenschaften der ersten Membran zurückzuführen sind. Die zweite Membran sollte nicht wasserdicht sein. Ist die zweite Membran wasserdurchlässig, so fördert dies den Fluss von Wundsekreten durch den Membranverbund, so dass die Mukosagewebezellen des Gewebetransplantat-konstruktes sowie die Zellen, die an das Gewebetransplantatkonstrukt angrenzen oder in dieses eingedrungen sind, von dem Wundsekreten erreicht werden können. Die Flüssigkeit-Durchgängigkeit der zweiten Membran verhindert zusätzlich die Bildung von Ödemen und die damit verbundene Trennung des Implantats vom Wundbett.

Das erfindungsgemäße Gewebetransplantatkonstrukt besitzt eine hohe Biokompatibilität und entspricht den gesetzlichen Bestimmungen an Medizinprodukte.

Bevorzugt ist die erste Membran equinen oder bovinen Ursprungs. Neben Kollagen, insbesondere Kollagen-Fasern, kann die erste Membran weitere Bestandteile wie beispielsweise Fibronektion enthalten. Geeignete erste Membranen sind die unter dem Handelnamen "TissuFoil" (Hersteller: Baxter Deutschland GmbH, DE) vertriebenen equinen kollagenhaltigen Membranen sowie die unter dem Handelsnamen "Matriderm" (Hersteller: Dr. Suwelack Skin & Health Care AG, DE) vertriebenen Kollagen/Fibronektion-Membranen.

Eine bevorzugte zweite Membran ist eine Membran, die von Warmblüter oder einem Menschen stammt. Stärker bevorzugt ist die zweite Membran eine Membran porzinen Ursprungs. Eine besonders geeignete zweite Membran ist eine porzine Dünndarm-Submukosa, insbesondere eine lyophilisierte porzine Dünndarm-Submukosa. Die zweite Membran kann neben Kollagen, insbesondere Kollagen-Fasern, weitere Bestandteile wie Glykoproteine, Proteoglykane und Glykosaminoglykane enthalten.

Die erste Membran kann ein- oder mehrlagig sein. Ebenso kann die zweite Membran ein- oder mehrlagig sein. Insbesondere kann die zweite Membran ein- bis vierlagig sein. Unter einer Membran wird hier insbesondere ein flächiges poröses Gebilde verstanden.

Der Membranverbund besteht aus Membranen biologischen und nicht synthetischen Ursprungs. Damit ist der Membranverbund des erfindungsgemäßen Gewebetransplantatkonstruktes im Gegensatz zu Gefäßtransplantaten (sogenannte "vascular grafts") vollständig abbaubar, was eine Kalzifizierung oder Abstoßung des Gewebetransplantatkonstruktes in der Zeit nach der Implantation verhindert. Die Anwesenheit von vitalen Zellen auf der Membran erlaubt die Generierung vom neuen Gewebe durch körpereigene Zellen. Hiermit erübrigt sich die Notwendigkeit der dauerhaften Haltbarkeit der Prothese bzw. des Trägermaterials.

Die Erfinderin der vorliegenden Erfindung hat überraschenderweise festgestellt, dass sich ein Membranverbund aus den beiden kollagenhaltigen ersten und zweiten Membranen durch Verpressen der ersten und zweiten Membranen und/oder durch Vernetzen, insbesondere durch Photovernetzen, der ersten und zweiten Membranen herstellen lässt. Ein so erhaltener Membranverbund hat eine ausreichende mechanische Stabilität, so dass er für die Herstellung eines Gewebetransplantatkonstruktes verwendet werden kann, der insbesondere Ersatz für Epithelgewebe eines tierischen und oder menschlichen Organs eingesetzt werden kann. Der erfindungsgemäße Membranverbund und ein unter Verwendung des erfindungsgemäßen Membranverbundes hergestelltes Gewebetransplantatkonstrukt sind daher insbesondere für die Rekonstruktion von Epithelgewebe geeignet. Besonders geeignet ist das erfindungsgemäße Gewebetransplantatkonstrukt für die Rekonstruktion eines Harnorgans, insbesondere der Harnblase, des Harnleiters oder der Harnröhre, sowie des Ösophagus, der Augenoberfläche oder oraler Gewebedefekte.

Die erfindungsgemäß vorgesehen Mukosagewebezellen sind autologe orale Mukosagewebezellen. Aufgrund der Verwendung autologer Mukosagewebezellen sind die erfindungsgemäßen Gewebetransplantatkonstrukte insbesondere für die Reparatur und/oder den Ersatz von epithelialem Gewebe geeignet.

Besonders geeignete autologe orale Mukosagewebezellen sind Mundschleimhautgewebezellen. Mundschleimhautgewebezellen verfügen über ein starkes Proliferationspotential und sind über relativ nicht-invasive Biopsien zugänglich, was das Mundschleimhautepithelgewebe zu einer attraktiven Zellquelle für autologe Therapien macht.

Einzelheiten zu den autologen oralen Mukosagewebezellen können dem nachfolgenden Abschnitt "Autologen oralen Mukosagewebezellen" entnommen werden.

Erfindungsgemäß ist ferner ein Verfahren zur Herstellung eines Gewebetransplantatkonstruktes vorgesehen, das die Schritte
(a) Herstellen eines Membranverbundes, umfassend zumindest eine erste biologisch kompatible, kollagenhaltige Membran und eine zweite biologisch kompatible, kollagenhaltige Membran, wobei die erste Membran und die zweite Membran an ihren Flachseiten aneinander angrenzen und wobei die erste Membran equinen oder bovinen Ursprungs ist und die zweite Membran tierischen oder human Ursprungs ist und die zweite Membran anderen Ursprungs als die erste Membran ist, unter photochemishen Vernetzen der ersten und der zweiten Membran; und
(b) Ausbilden ein oder mehrerer Schichten von autologen oralen Mukosagewebezellen auf einer oder beiden äußeren Flachseiten des Membranverbundes
umfasst.

Schritt (a) dieses Verfahrens umfasst dabei vorzugsweise die folgenden Teilschritte:
(a1) Bereitstellen von zwei ersten Membranen und Überführen der ersten Membranen in einen gequollenen Zustand;
(a2) Bereitstellen einer zweiten Membran und Lyophiliseren der zweiten Membran;
(a3) Anordnen der zweiten Membran zwischen den beiden ersten Membranen; und
(a4) Herstellen eines Verbundes aus den ersten Membranen und der zweiten Membran.

Bevorzugt wird der Membranverbund durch Verpressen und Vernetzen der ersten und der zweiten Membranen hergestellt. Das Verpressen erfolgt vorzugsweise unter einem Druck von 5 bis 5000 kN/cm², stärker bevorzugt 10 bis 1000 kN/cm² und noch stärker bevorzugt bei 50 bis 150 kN/cm² und am stärksten bevorzugt bei 100 kN/cm². Bei Drücken, die geringer als 5 kN/cm² sind, kann möglicherweise kein hinreichend fester Verbund der Membranen erreicht werden, während bei Drücken über 5000 kN/cm² die Membranen, insbesondere deren Gerüst- und Porenstruktur beschädigt werden können.

Es ist bevorzugt, dass Verpressen bei einer Temperatur durchzuführen, die gegenüber der Umgebungstemperatur leicht erhöht ist, bevorzugt bei 25 bis 50 °C, stärker bevorzugt bei 35 bis 40 °C. Die leicht erhöhte Temperatur fördert die Beweglichkeit der Kollagenfasern, ohne deren dreidimensionalen Struktur zu ändern. Das Verpressen wird vorzugsweise über einen Zeitraum von 10 min bis 2 h, stärker bevorzugt 0,5 bis 1,5 h und besonders bevorzugt 3 bis 13 Minuten durchgeführt.

Zusätzlich zum Verpressen wird der Membranverbund durch photochemischen Behandlung unterzogen, um eine Vernetzung der Kollagenfasern der ersten und zweiten Membranen zu erreichen. Das Verfahren zur photochemischen Vernetzung von Kollagenfasern ist insbesondere aus der Ophtalmologie zur Behandlung von Keratokonus bekannt. Das dortige Verfahren der Hornhaut-Kollagenvemetzung besteht aus der Photopolymerisation von Stromafasern durch die kombinierte Wirkung einer lichtsensibilisierenden Substanz (Riboflavin oder Vitamin B2) und Ultraviolett-A-Strahlen (UVA). Die Photopolymerisation erhöht die Steifigkeit von Hornhautkollagen und seine Beständigkeit gegen Keratektasie (siehe beispielsweise: Corneal collagen cross-linking with riboflavin und ultraviolet-A light for keratoconus: Results in Indian eyes. Agrawal V. Indian Journal of Ophtalmology. 2008:57(2).111-114).

Die photochemische Vernetzung wird vorzugsweise mit sichtbarem Licht durchgeführt. Vorzugsweise hat das Licht eine Wellenlänge von 380 bis 600 nm, stärker bevorzugt von 425 bis 525 nm, besonders bevorzugt 475 nm. Die Vernetzung wird vorzugsweise über einen Zeitraum von 10 min bis 2 h, besonders bevorzugt 0,5 bis 1,5 h durchgeführt.

### Autologe orale Mukosagewebezellen

### a) Verwendung autologer Mundschleimhautzellen bei der urologischen Rekonstruktion

Urethra- und Ureterstrikturen sind Verengungen des Organs, verursacht durch Entzündung, Narbengewebe, Dauerkatheter, Instrumentierung, externes Trauma, Operationen. In diesem Fall ersetzt Narbengewebe das normale Urethra- oder Ureterepithelgewebe. Offene Urethroplastik und Ureteroplastik werden als die Goldstandardbehandlung einer Urethra- und Ureterstriktur angesehen. Mundschleirnhauttransplantate sind als der vielversprechendste Ersatz in urologischen Organen anerkannt. Donorstellenmorbidität an oralen Stellen ist jedoch ein Hauptanliegen.

Gewebetechnologie bei urologischer Rekonstruktion hat seit dem ersten Bericht einer In-vitro-Kultur von Übergangszellepithel von Bunge 1955 einen langen Weg zurückgelegt. Natürlich sollte zum Erhalt einer erfolgreichen Substitutions-Urethroplastik ein auf Gewebetechnologie basierendes Produkt über eine Matrix verfügen, die biokompatibel und robust und unter Zug vemähbar ist, und gleichzeitig die optimale Abgabe von Zellen an den Ort der Urethroplastik und auch die adäquate Fixation des Transplantates an der Implantationsstelle und Wundstabilisierung ermöglicht. Während kultivierte Mundschleimhautzellen optimale Zellkandidaten für auf Gewebetechnologie basierende urologische Organe darstellen, werden in klinischen und experimentellen Anordnungen mehrere Materialien, sowohl organische als auch synthetische, zur Bereitstellung eines Urethra- und Ureterersatzes verwendet; diese Materialien enthalten azelluläre Blasenmatrizen, azelluläre Schweine-Dünndarm-Submukosa (SIS), Gewebe aus Dexon, Kollagenmatrizen und Polytetrafluorethylen (GORE-TEX) *(siehe:* Romagnoll G et al., Onestep treatment of proximal hypospadias by the autologous graft of cultured urethral epithelium. Journal of Urology.1993:150(4).1204 - 1207*...* El-Kassaby A W et al., Urethral stricture repair with an off-the-shelf collagen matrix. Journal of urology. 2003: 169(1). 170-173*....* Badylak SF et al., The extrazellular matrix as a biologic scaffoldl material. Biomaterials. 2007:28.3587-3593*).* Diese Materialien hatten in der Regel eingeschränkten Erfolg aufgrund mechanischer, struktureller oder Biokompatibilitätsprobleme. Kürzlich ist gezeigt worden, das nicht mit Zellen besiedelten SIS aufgrund ihrer mechanischen Reißfestigkeit im Wesentlichen vielversprechender sind, gute Ergebnisse erzielte man jedoch nur bei Patienten mit Defekten an kurzen *Urethrasegmenten (siehe:* Bhargava et al., Gewebe-Engineered Buccal Mucosa Urethroplasty-Clinical Outcomes. European Urology.2008:53(6).1263-1271*).* Bei Patienten mit längerer oder Anurie war die Reepithelialisierung nicht mit Zellen besiedelten Proteingerüste nie vollständig erfolgreich *(siehe:* Palminteri E et al., Small intestinal submucosa (SIS) graft urethroplast: shortterm results. European Urology. 2007:51(6).1695-1701 Fiala R et al.,. Porcine small intestinal submucosa graft for repair of anterior urethral strictures. European Uroloy. 2007:51(6).1702-1708*).* Bhargava et al., berichteten über den klinischen Verlauf einer Technik für auf Gewebetechnologie basierender autologer Wangenschleimhaut auf einer deepidermisierten, sterilisierten Spenderhautmatrix in der Substitutions-Urethroplastik. Das in dieser Studie verwendete Proteingerüst (deepidermisierte Dermis) wurde von abgeschirmten Organspendern über die National Blood Service Skin Bank erhalten. Das Proteingerüst erfordert jedoch Leichenmaterial, das knapp ist. Außerdem waren die Studienergebnisse der Urethroplastik beim Mann nicht optimal. In dieser Veröffentlichung wurde eine frühe Entzündungsreaktion gegen das Proteingerüst als ein anderer Grund für Transplantatkontraktion und Fibrose diskutiert. Tatsächlich wird eine Proteingerüst-artige deepidermisierte Dermis in vivo nicht in kurzer Zeit (1 bis 2 Wochen) zersetzt, und so kann im Ergebnis eine starke Entzündungsreaktion im Körper induziert werden.

### b) Verwendung autologer Mundsehleimhautzellen für die Augenoberflächenrekonstruktion

Eine schwere Erkrankung der Augenoberfläche, die durch Zustände wie das Stevens-Johnson-Sydrom und okulares Narbenpemphigoid entsteht, ist ein potentiell zestörender Zustand mit signifikanter visueller Morbidität. In solchen Fällen werden die Homhautepithelstammzellen im Limbus zerstört, was zur Invasion der Homhautoberfläche durch umgebende Konjunktiva, Neovaskularisation, chronische Entzündung, Einwuchs von Fasergewebe und Stromavernarbung führt. Die herkömmliche Hornhauttransplantation bei diesen Patienten ist mit schrecklichen Ergebnissen verbunden. Alternative Verfahren wie die Transplantation kultivierter Hornhautepithelstammzellen wurden demonstriert. Auf diese Weise erhielten Patienten mit unilateraler Hornhautschädigung Transplantate von kultivierten Hornhautepithelstammzellen, die man aus dem gesunden kontralateralen Auge erhielt. Die Gesundheit des Auges ist jedoch ein Hauptanliegen. Bei Patienten mit bilateraler Augenschädigung ist die Transplantation kultivierter Hornhautepithelstammzellen von Spenderleichen oder einem Lebendspenderauge erforderlich. Trotz einigen Erfolges stellt die immunologische Abstoßung und mikrobielle Infektion als Folge einer immunosuppressiven Therapie nach allogener Transplantation nach wie vor eine Herausforderung dar. Im Kontext der regenerativen Medizin stellt die Transplantation kultivierter Schleimhautepithelstammzellschichten, erzeugt aus autologen Zellquellen, eine entwicklungsfähige Alternative in Fällen bilateraler Augenschädigung dar, die die Verwendung autologer Homhautepithelstammzellen hinfällig macht. Mundschleimhautzellen haben Aufmerksamkeit als eine Zellquelle erregt, und in Tier- und einleitende Menschen-Pilotstudien wurden positive Ergebnisse erhalten. Dieses Verfahren verringert das Risiko der Transplantatabstoßung und die Notwendigkeit von Langzeit-Steroiden oder -Immunosuppression. *(siehe:* Midterm results on ocular surface reconstructoin using cultivated autolog oral mucosa epithelial transplantaion. Inalomi T, Nakammura T, Koizumi N,... American Journal of ophtalmology. 2006:141(2). 267-276*.* The use of autolgous serumirt the development of corneal und oral epithelial equivalents in patients with Steven .lohnson Syndrome. Nakamura T, Ang L, Rigby H, Sekiyama E,... Investigative ohtalmology und visual science. 2006:47(3). 909-914*).* Das derzeit bevorzugte Verfahren zur Kultivierung von Hornhaut- oder oralen Epithelzellen erfordert die Verwendung mechanisch instabiler Materialien, meistens einer amniotischen Membran (siehe: *Inatomi et al., Nakamura et al., oben).* Die Verwendung einer amniotischen Membran erfordert auch die allogene Plazenta von Frauen, die einen Kaiserschnitt hatten, wobei es hier an Material mangelt. Dieses Problem besteht auch bei anderen vorgeschlagenen Mundschleimhautzellkonstrukten, wie EVPOME, welches ebenso Leichenmaterial erfordert *(siehe: 3* Clinical und Histopathological Analysis of Healing Process of Intraoral Reconstruction with ex vivo Produced Oral Mucosa Equivalent. HOTTA T, YOKOO 5, TERASHI H. Kobe Journal of medical science. 2007: 53(1).1-14*).*

### c) Verwendung autologer Mundschleinthautzellen für die Speiseröhrenrekonstruktion

Die azellulären Matrizen wurden für die Ösophagoplastik in Tiermodellen verwendet. Diese führte jedoch nicht zu einer vollständigen Epithelbildung. Für eine bessere Rekonstruktion ist daher eine zelluläre Komponente erforderlich. In Tiermodellen zeigte die Verwendung von Mundschleimhautepithelzellen auf azellulärer Dünndarm-Submukosa viel versprechende Ergebnisse bei der Rekonstruktion von kurzen Ösophagusdefekten von etwa 5 cm. Aufgrund der lyophilisierten Form der Dünndarm-Submukosa ist eine mehrlagige Kultur von Zellen auf längeren Segmenten der Membran jedoch nicht immer möglich (siehe: Grafts of Porcine Small Intestinal Submucosa with Cultured Autolog Oral Mucosa Epithelial Cells for Esophageal Repair in a Canine Model. Wei R, Tan B, Tan M, Luo J, Deng L,... Experimental biology und medicine. 2009:234. 453-461*).* Auch andere berichtete Membranen für die Ösophagoplastik, bestehend aus Kollagen, sind für die Verwendung am Menschen mechanisch viel zu instabil (siehe: Esophagus Tissue engineering: in vitro generation of esophageal epithelial cells sheets und viability on scaffold. Saxena A Ainoedhofer H,Höllwarth M. Journal of pediatric surgery. 2009:44. 896-901*).* Weitere synthetische Materialien für die Ösophagoplastik wie Poly(1-milchsäure) (PLLA), Poly(milch-co-glycol)säure (75 : 25) (PLGA75), Poly(milch-co-glycol)säure (50 : 50) (PLGA50) und Polycaprolacton/Poly(1-milchsäure) (50 : 50) (PCL/PLLA) haben sich für die Gewebetechnologie in Bezug auf die Speiseröhre als ungeeignet erwiesen (siehe: Esophageal epithelial cell interaction with synthetic und natural scaffolds for tissue engineering. Beckstead Ba, Pan 5, Bhrany A, Bratt-Leal A, ... Biomaterials.2005:26(31).6217-6228*)*

### d) Verwendung autologer Mundschleimhautzellen für die Hautrekonstruktion (Verbrennungen)

Orale Keratinozyten haben mehrere einzigartige Merkmale, die Vorteile gegenüber epidermalen (Haut) Keratinozyten bieten können. Orale Keratinozyten weisen eine höhere Proliferationsrate und eine geringere Enddifferenzierungsrate als epidermale Keratinozyten auf. Aus diesem Grund können relativ kleine Spenderstellen mittels Ex-vivo-Expansion ausreichend Zellmasse zur Abdeckung viel größerer Wunden liefern. Außerdem sekretieren orale Keratinozyten pro-angiogene Faktoren, wie VEGF und IL8, die ihre schnelle Integration an den Transplantationsstellen fördert. Daher haben sich Mundschleimhautzellen als geeignete Kandidaten für die Hautrekonstruktion erwiesen (siehe: Development of a tissue-engineered human oral mucosa equivalent based on an acellular allogeneic dermal matrix: A preliminary report of clinical application to burn wounds. Takuya L, Takami V. Yamaguchi R, Shimazaki 5,... Scandinavian Journal of Plastic und Reconstructive Surgery und Hand Surgery. 2005: 39(3).138-146*.* Der Nachteil dieses Konstruktes ist, wie oben erwähnt, die Anwendung von humanen Leichenmaterialien.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Darstellung einer ersten Ausführungsform eines erfindungsgemäßen Membranverbundes im Querschnitt;
- Fig. 2: eine schematische Darstellung einer ersten Ausführungsform eines erfindungsgemäßen Gewebetransplantatkonstruktes;
- Fig. 3: eine schematische Darstellung einer zweiten Ausführungsform eines erfindungsgemäßen Gewebetransplantatkonstruktes;
- Fig. 4: eine schematische Darstellung einer zweiten Ausführungsform eines erfindungsgemäßen Membranverbundes im Querschnitt; und
- Fig. 5: eine schematische Darstellung einer dritten Ausführungsform eines erfindungsgemäßen Membranverbundes im Querschnitt.

### Beispiele

### Beispiel 1: Herstellung eines Membranverbundes

Es wurde ein Membranverbund hergestellt, der zwei erste Membranen und eine zweite Membran aufwies, wobei die zweite Membran zwischen den beiden ersten Membranen angeordnet war.

Als erste Membran wurden zwei biologisch abbaubare equine Kollagen-Membranen (Handelsname "TissuFoil" der Baxter Deutschland GmbH, DE) oder zwei bovine Kollagen/Fibronektin-Membranen (Handelsname "Matriderm" der Dr. Suwelack Skin & Health Care AG, DE) verwendet. Diese Membranen sind zertifizierte Medizinprodukte, und dürfen somit in Patienten angewendet werden.

Als zweite Membran wurde eine lyophilsierte porzine Dünndarm-Submukosa verwendet, die insbesondere Kollagen, Glykoproteine, Proteoglykane und Glykosaminoglykane enthielt.

Die beiden ersten Membranen wurden für 24 Stunden in phosphat-gepufferter Kochsalzlösung platziert, wodurch sie in eine gequollene und poröse Form überführt wurde. Danach wurde die zweite Membran zwischen den beiden ersten Membranen platziert. Das so erhaltene dreilagige Gebilde wurde bei einer Temperatur von 35 bis 40 °C unter einem Druck von 100 kN/cm² unter Erhalt des Membranverbundes komprimiert (Zeitdauer der Kompression: 5 Minuten). Anschließend wurde der Membranverbund für eine Stunde mit 5%iger Vitamin- (Riboflavin) -Lösung getränkt. Danach wurde der Membranverbund eine Stunde mit sichtbarem Licht, das eine Wellenlänge von 475 nm aufwies, behandelt, um die Steifigkeit des Membranverbundes zu erhöhen und dessen Kontraktion nach seiner Implantation zu verringern.

Es wurden Membranverbunde mit folgendem Aufbau erhalten:
1.1: Equine Membran / porzine Dünndarm-Submukosa/ equine Membran
1.2: Bovine Membran / porzine Dünndarm-Submukosa/ bovine Membran

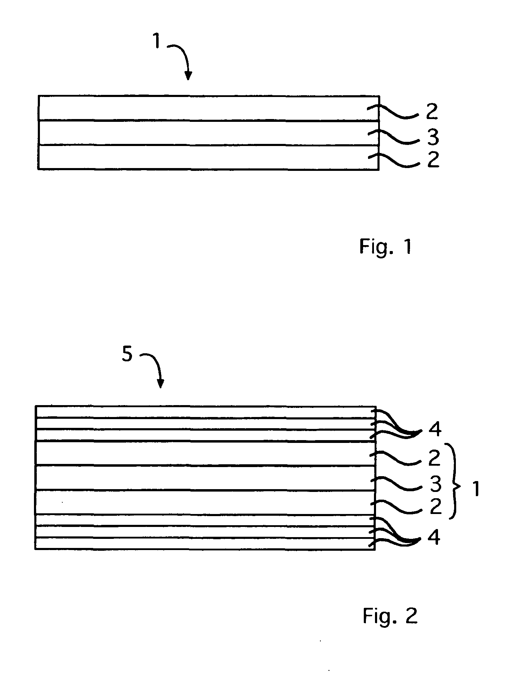

Der Aufbau des Membranverbundes 1.1 und der Aufbau des Membranverbundes 1.2 sind schematisch in Fig. 1 im Querschnitt gezeigt. Der dort dargestellte Membranverbund 1 weist zwei erste Membranen 2 und eine zweite Membran 3 auf. Die zweite Membran 3 ist zwischen den beiden ersten Membranen 2 sandwich-artig angeordnet.

### Beispiel 2: Herstellung eines erfindungsgemäßen Gewebetransplantatkonstruktes zur Verwendung als Schleimhauttransplantat

Gemäß Beispiel 1 hergestellter Membranverbünde (Membranverbund 1.1 oder Membranverbund 1.2) wurden zur Herstellung erfindungsgemäßer Gewebetransplantatkonstrukte, die als Schleimhauttransplantate verwendet werden sollen, mit Mukosakeratinozyten besät. Zu diesem Zweck wurde eine Biopsieprobe mit einem Durchmesser von 2 bis 4 mm aus der Wangenschleimhaut von 40 Patienten entnommen. Des Weiteren wurden 30 ml autogenes Serum aus einer venösen Vollblutprobe dieser Patienten extrahiert. Die Primärkulturen werden in Dulbecco's Modified Eagle Medium und Nährstofffaktor F 12 (Gibco Co., Eggenstein, Deutschland), welche die üblichen (siehe Lauer G, Schimming R, Klinische Anwendung von im Tissue Engineering gewonnenen autologen Mundschleimhauttransplantaten. Mund Kiefer Gesichtschirurgie.2002. 6:379-393) Additive und autogenes Serum enthielten, drei Wochen in bekannter Weise (siehe Lauer G, Schimming R, Klinische Anwendung von im Tissue Engineering gewonnenen autologen Mundschleimhauttransplantaten. Mund Kiefer Gesichtschirurgie.2002. 6:379-393) inkubiert. Anschließend wurde zur Konstruktion von Schleimhauttransplantaten, die mehrere Schichten von Mundschleimhautzellen aufweisen, auf dem in Beispiel 1 erhaltenen Membranverbund subkultiviert.

Die inkubierten Schleimhautgewebszellen wurden auf beide Flachseiten des Membranverbundes aufgebracht und dort kultiviert. Nach 48 Stunden zeigte die Zellverteilungsanalyse mit MTT-Farbstoff eine Membranabdeckung von > 90 % auf beiden Seiten des Membranverbundes. Assays hinsichtlich der Lebensfähigkeit der Zellen mit Calcein/Ethidium-Bromid-Fluoreszenzfarbstoffen zeigten eine Zelllebensfähigkeit auf der Membran von > 90 %. Überdies zeigten > 30 % der Zellen eine positive Reaktion mit Bromdesoxyuridin (BrdU) und weisen daher Proliferationsfähigkeit auf.

Es wurden Gewebetransplantatkonstrukte mit folgendem Aufbau erhalten:
2.1: Ein oder mehrere Schichten aus Mukosagewebezellen / equine Membran / porzine Dünndarm-Submukosa/ equine Membran / ein oder mehrere Schichten aus Mukosagewebezellen
2.2: Ein oder mehrere Schichten aus Mukosagewebezellen / bovine Membran / porzine Dünndarm-Submukosa/ bovine Membran / ein oder mehrere Schichten aus Mukosagewebezellen

In Fig. 2 ist der Aufbau eines gemäß Beispiel 2 hergestellten Gewebetransplantatkonstruktes 5 schematisch im Querschnitt gezeigt. Die äußeren Flachseiten des in Fig. 1 gezeigten Membranverbundes 1, also bezogen auf Fig. 1 dessen Oberseite und dessen Unterseite, sind mit mehreren Schichten 4, die aus Mukosagewebezellen gebildet sind, bedeckt.

### Beispiel 3

In derselben Weise wie in Beispiel 2 wurden die beiden Flachseiten der gemäß Beispiel 1 erhaltenen Membranverbünde mit unterschiedlichen Zellkulturen besät. Dabei wurden eine äußere Flachseite des jeweiligen Membranverbundes Keratinozyten kultiviert, während auf der anderen Flachseite des Membranverbundes ein Gemisch aus Mundgewebefibroblasten und Mukosagewebe-Endothelzellen kultiviert wurde (Quelle der Zellen: Munschleimhautgewebe-Biopsie; Mischungsverhältnis zwischen den Mundfibroblasten und den Endothelzellen 1 : 3.) Diese Mischpopulation wurde im Anschluss an die Keratinozytenbesiedelung der einen Flachseite des Membranverbundes nach 30 Minuten auf die andere Flachseite des Membranverbundes aufgebracht. Im Übrigen entspricht die Verfahrensweise zur Kultivierung der Zellen der von Beispiel 2.

Es wurden Gewebetransplantatkonstrukte mit folgendem Aufbau erhalten:
2.1: Ein oder mehrere Schichten aus Keratinozyten / equine Membran / porzine Dünndarm-Submukosal equine Membran / ein oder mehrere Schichten aus einem Gemisch aus Mundfibroblasten und Endothelzellen
2.2: Ein oder mehrere Schichten aus Keratinozyten / bovine Membran / porzine Dünndarm-Submukosal bovine Membran / ein oder mehrere Schichten aus Gemisch aus Mundfibroblasten und Endothelzellen

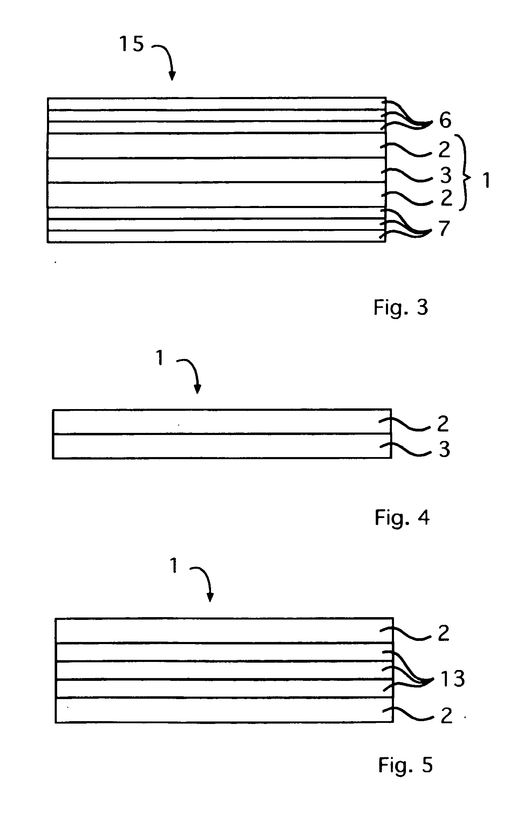

In Fig. 3 ist der Aufbau eines gemäß Beispiel 3 hergestellten Gewebetransplantatkonstruktes 15 schematisch im Querschnitt gezeigt. Die obere Flachseite 1 des in Fig. 1 gezeigten Membranverbundes 1 ist mit mehreren Schichten 6, die aus Keratinozyten gebildet sind, bedeckt. Die untere Flachseite des Membranverbundes 1 ist mit mehreren Schichten 7, die aus einem Gemisch aus Mundfibroblasten und Endothelzellen gebildet sind, bedeckt.

### Vergleichsbeispiel 1

Zu Vergleichszwecken wurden die in Beispiel 2 und Beispiel 3 beschriebenen Zellkulturen auf Vergleichsmembranen aufgetragen. Als Vergleichsmembranen wurden verwendet: i) porzine Dünndarm-Submukosa; ii) eine equine Membran (Handelsname: "TissuFoil") und iii) eine bovine Membran (Handelsname: "Matriderm"). Die Vergleichsmembranen entsprechen den in Beispiel 1 verwendeten ersten oder zweiten Membranen, außer dass keine Verbundmembran hergestellt wurde. Die Kultivierungsbedingungen der Zellen waren dieselben wie in Beispiel 2. Es wurde festgestellt, dass das Zellwachstum auf einer unbehandelten equinen Membran (TissuFoil) oder bovinen Membran (Matriderm) Ähnlichkeit zu dem in Beispiel 2 beschriebenen Kultivierungsprozess auf einem erfindungsgemäßen Membranverbund zeigte, während bei der Verwendung von Dünndarm-Submukosa als Membran eine geringere Membranabdeckung (etwa 50 %) mit einer Zelllebensfähigkeit von etwa 70 % und einer Proliferationskapazität von < 10 % zu beobachten war.

Es wurden folgende Vergleichskonstrukte erhalten:
i) Ein oder mehrere Schichten aus Mukosagewebezellen / porzine Dünndarm-Submukosa / ein oder mehrere Schichten aus Mukosagewebezellen
ii) Ein oder mehrere Schichten aus Mukosagewebezellen / equine Membran / ein oder mehrere Schichten aus Mukosagewebezellen
iii) Ein oder mehrere Schichten aus Mukosagewebezellen / bovine Membran / ein oder mehrere Schichten aus Mukosagwebezellen

### Beispiel 4 und Vergleichsbeispiel 2: Mechanische Eigenschaften der erfindungsgemäßen Gewebetransplantatkonstrukte

Die Stabilität und die Zugfestigkeit der in Beispiel 2 und Vergleichsbeispiel 1 hergesellten Gewebetransplantatkonstrukte wurden 48 Stunden nach der Zellaussaat untersucht. Es wurde festgestellt, dass die in Beispiel 2 hergestellten, erfindungsgemäßen Gewebetransplantatkonstrukte nicht rissen und gleichzeitig ausgezeichnete Vemähbarkeit, Zugfestigkeit, Fadenausziehfestigkeit und Knotenapplikationsfestigkeit zeigten. Die Vergleichskonstrukte mit Dünndarm-Submukosa, die gemäß Vergleichsbeispiel 1 hergestellt wurden, zeigten eine ähnliche mechanische Stabilität. Vergleichskonstrukte mit den equinen Membranen (TissuFoil) und bovinen Membranen (Matriderm), die ebenfalls gemäß Vergleichsbeispiel 1 hergestellt wurden, zeigten geringe mechanische Stabilität und rissen leicht unter Zug, bei der Fadenausziehbehandlung oder der Knotenapplikation.

### Beispiele 5 und 6: Aufbau von weiteren Membranverbünden

Der in Fig. 4 gezeigte Membranverbund 1 weist im Gegensatz zu dem in Fig. 1 gezeigten Membranverbund nur eine erste Membran 2 auf. Damit ist nur eine Flächenseite der zweiten Membran bedeckt. Der in Fig. 5 gezeigte Membranverbund 1 weist im Gegensatz zu dem in Fig. 1 gezeigten Membranverbund eine mehrlagige zweite Membran 13 auf. Abgesehen davon entsprechen der Aufbau und die Herstellung dieser Membranverbünde denen von Beispiel 1.

### Bezugszeichenliste

- 1: Membranverbund
- 2: Erste Membran
- 3: Zweite Membran
- 4: Schichten aus Mukosagewebezellen
- 5: Gewebetransplantatkonstrukt
- 6: Schicht aus Keratinozyten
- 7: Schicht aus einem Gemisch aus Mundfibroblasten und Endothelzellen
- 13: mehrschichtige zweite Membran
- 15: Gewebetransplantatkonstrukt

## Patentansprüche

1. Gewebetransplantatkonstrukt zur Rekonstruktion eines menschlichen oder tierischen Organs, umfassend
(a) einen photochemisch vernetzten Membranverbund (1), umfassend zumindest eine erste biologisch kompatible, kollagenhaltige Membran (2) und zumindest eine zweite biologisch kompatible, kollagenhaltige Membran (3), wobei die erste Membran (2) und die zweite Membran (3) an ihren Flachseiten aneinander angrenzen und wobei die erste Membran (2) equinen oder bovinen Ursprungs ist und die zweite Membran (3) tierischen oder humanen Ursprungs ist und die zweite Membran (3) anderen Ursprungs als die erste Membran (2) ist; und
(b) ein oder mehrere Schichten von autologen oralen Mukosagewebezellen (4, 5, 6) auf einer oder beiden äußeren Flachseiten des Membranverbundes (1).

2. Gewebetransplantatkonstrukt nach Anspruch 1, **dadurch gekennzeichnet, dass** der Membranverbund (1) zwei erste Membranen (2) und eine zweite Membran (3) umfasst, wobei die zweite Membran (3) zwischen den beiden ersten Membranen (2) angeordnet ist.

3. Gewebetransplantatkonstrukt nach Anspruch 1 oder Anspruch 2, **dadurch gekennzeichnet, dass** die erste Membran (2) equinen oder bovinen Ursprungs ist und die zweite Membran (3) porzinen Ursprungs ist.

4. Gewebetransplantatkonstrukt nach einem der vorstehenden Ansprüche, **dadurch gekennzeichnet, dass** die erste Membran (2) neben Kollagen auch Fibronectin enthält.

5. Gewebetransplantatkonstrukt nach einem der vorstehenden Ansprüche, **dadurch gekennzeichnet, dass** die ersten und zweiten Membranen (2, 3) des Membranverbundes (1) miteinander verpresst und/oder vernetzt sind.

6. Membranverbund für ein Gewebetransplantatkonstrukt, umfassend zumindest eine erste biologisch kompatible, kollagenhaltige Membran (2) und eine zweite biologisch kompatible, kollagenhaltige Membran (3), wobei die erste Membran (2) und die zweite Membran (3) an ihren Flachseiten aneinander angrenzen und wobei die erste Membran (2) equinen oder bovinen Ursprungs ist und die zweite Membran (3) tierischen oder humanen Ursprungs ist und die zweite Membran (3) anderen Ursprungs als die erste Membran (2) ist, wobei die erste und die zweite Membran photochemisch vernetzt sind.

7. Membranverbund nach Anspruch 6, **dadurch gekennzeichnet, dass** er zwei erste Membranen (2) und eine zweite Membran (3) umfasst, wobei die zweite Membran (3) zwischen den beiden ersten Membranen (2) angeordnet ist.

8. Membranverbund nach Anspruch 6 oder Anspruch 7, **dadurch gekennzeichnet, dass** die ersten und zweiten Membranen (2, 3) des Membranverbundes (1) miteinander verpresst und vernetzt sind.

9. Verfahren zur Herstellung eines Gewebetransplantatkonstruktes nach einem der Ansprüche 1 bis 5, umfassend
(a) das Herstellen eines Membranverbundes (1), umfassend zumindest eine erste biologisch kompatible, kollagenhaltige Membran (2) und eine zweite biologisch kompatible, kollagenhaltige Membran (3), wobei die erste Membran (2) und die zweite Membran (3) an ihren Flachseiten aneinander angrenzen und wobei die erste Membran (2) equinen oder bovinen Ursprungs ist und die zweite Membran (3) tierischen oder humanen Ursprungs ist und die zweite Membran (3) anderen Ursprungs als die erste Membran ist, unter photochemischen Vernetzen der ersten und der zweiten Membran; und
(b) das Ausbilden einer oder mehrerer Schichten (4) von autologen oralen Mukosagewebezellen auf einer oder beiden äußeren Flachseiten des Membranverbundes (1).

10. Verfahren nach Anspruch 9, **dadurch gekennzeichnet, dass** Schritt (a) die Schritte
(a1) Bereitstellen von zwei ersten Membranen (2) und Überführen der ersten Membranen (2) in einen gequollenen Zustand;
(a2) Bereitstellen einer zweiten Membran (3) und Lyophiliseren der zweiten Membran (3);
(a3) Anordnen der zweiten Membran (3) zwischen den beiden ersten Membranen (2); und
(a4) Herstellen eines Verbundes (1) aus den ersten Membranen (2) und der zweiten Membran (3)
umfasst.

11. Verfahren nach Anspruch 9 oder Anspruch 10, **dadurch gekennzeichnet, dass** der Membranverbund (1) durch Verpressen und Vernetzen der ersten und der zweiten Membranen (2, 3) hergestellt wird.

12. Verfahren nach einem der Ansprüche 9 bis 11, **dadurch gekennzeichnet, dass** die autologen oralen Mukosagewebezellen auf eine oder beide äußere Flachseiten des Membranverbundes (1) aufgebracht und dort unter Ausbildung einer oder mehrerer Schichten (4) kultiviert werden.

## Claims

1. A tissue transplant construct for the reconstruction of a human or animal organ comprising
(a) a photo-chemically cross-linked membrane composite (1) comprising at least a first biocompatible, collagen-containing membrane (2) and at least a second biocompatible, collagen-containing membrane (3), wherein the first membrane (2) and the second membrane (3) are adjacent to each other at their flat sides, and wherein the first membrane (2) is of equine or bovine origin and the second membrane (3) is of animal or human origin and the second membrane (3) is of another origin than the first membrane (2); and
(b) one or more layers of autologous oral mucosa tissue cells (4, 5, 6) on one or both outer flat sides of the membrane composite (1).

2. The tissue transplant construct according to claim 1, **characterized in that** the membrane composite (1) comprises two first membranes (2) and one second membrane (3), wherein the second membrane (3) is arranged between the two first membranes (2).

3. The tissue transplant construct according to claim 1 or claim 2, **characterized in that** the first membrane (2) is of equine or bovine origin and the second membrane (3) is of porcine origin.

4. The tissue transplant construct according to anyone of the preceding claims, **characterized in that** the first membrane (2) in addition to collagen also contains fibronectin.

5. The tissue transplant construct according to anyone of the preceding claims, **characterized in that** the first and second membranes (2, 3) of the membrane composite (1) are compressed and/or cross-linked with each other.

6. A membrane composite for a tissue transplant construct comprising at least one first biocompatible, collagen-containing membrane (2) and a second biocompatible, collagen-containing membrane (3), wherein the first membrane (2) and the second membrane (3) are adjacent to each other at their flat sides, and wherein the first membrane (2) is of equine or bovine origin and the second membrane (3) is of animal or human origin and the second membrane (3) is of another origin than the first membrane (2), wherein the first and the second membrane are photo-chemically cross-linked.

7. The membrane composite according to claim 6, **characterized in that** it comprises two first membranes (2) and one second membrane (3), wherein the second membrane (3) is arranged between the two first membranes (2).

8. The membrane composite according to claim 6 or claim 7, **characterized in that** the first and second membranes (2, 3) of the membrane composite (1) are compressed and cross-linked with each other.

9. A method for the preparation of a tissue transplant construct according to anyone of claims 1 to 5 comprising
(a) the preparation of a membrane composite (1) comprising at least one first biocompatible, collagen-containing membrane (2) and a second biocompatible, collagen-containing membrane (3), wherein the first membrane (2) and the second membrane (3) are adjacent to each other at their flat sides, and wherein the first membrane (2) is of equine or bovine origin and the second membrane (3) is of animal or human origin and the second membrane (3) is of another origin than the first membrane, to photo-chemically cross-link the first and second membranes; and
(b) the formation of one or more layers (4) of autologous oral mucosa tissue cells on one or both of the outer flat sides of the membrane composite (1).

10. The method according to claim 9, **characterized in that** step (a) comprises the steps of
(a1) providing two first membranes (2) and converting the first membranes (2) into a swollen state;
(a2) providing a second membrane (3) and lyophilizing the second membrane (3);
(a3) arranging the second membrane (3) between the two first membranes (2); and
(a4) preparing a composite (1) of the first membranes (2) and the second membrane (3).

11. The method according to claim 9 or claim 10, **characterized in that** the membrane composite (1) is prepared by compressing and cross-linking the first and the second membranes (2, 3).

12. The method according to anyone of claims 9 to 11, **characterized in that** autologous oral mucosa tissue cells are applied onto one or both of the outer flat sides of the membrane composite (1) and there are cultured to form one or more layers (4).

## Revendications

1. Un produit de construction tissulaire transplantable destiné à la reconstruction d'un organe humain ou animal comprenant
(a) une membrane composite (1) à réticulation photochimique comprenant au moins une première membrane biocompatible contenant du collagène (2) et au moins une seconde membrane biocompatible contenant du collagène (3), la première membrane (2) et la seconde membrane (3) étant adjacentes l'une à l'autre par leurs faces planes, la première membrane (2) étant d'origine équine ou bovine et la seconde membrane (3) d'origine animale ou humaine et la seconde membrane (3) étant d'une autre origine que la première membrane (2) ; et
(b) une ou plusieurs couches de cellules de tissu muqueux autologues orales (4, 5, 6) sur l'une des deux ou sur les deux faces planes de la membrane composite (1).

2. Produit de construction tissulaire transplantable selon la revendication 1 **caractérisé en ce que** la membrane composite (1) comprend les deux premières membranes (2) et une seconde membrane (3), la seconde membrane (3) étant disposée entre les deux premières membranes (2).

3. Produit de construction tissulaire transplantable selon la revendication 1 ou la revendication 2 **caractérisé en ce que** la première membrane (2) est d'origine équine ou bovine et la seconde membrane (3) est d'origine porcine.

4. Produit de construction tissulaire transplantable selon l'une quelconque des revendications précédentes **caractérisé en ce que** la première membrane (2), en plus du collagène, contient également de la fibronectine.

5. Produit de construction tissulaire transplantable selon l'une quelconque des revendications précédentes **caractérisé en ce que** les premières et secondes membranes (2, 3) de la membrane composite (1) sont comprimées l'une contre l'autre et/ou réticulées l'une avec l'autre.

6. Membrane composite pour un produit de construction tissulaire transplantable comprenant au moins une première membrane biocompatible contenant du collagène (2) et une seconde membrane biocompatible contenant du collagène (3), la première membrane (2) et la seconde membrane (3) étant adjacentes l'une à l'autre par leurs faces planes, la première membrane (2) étant d'origine équine ou bovine et la seconde membrane (3) d'origine animale ou humaine et la seconde membrane (3) étant d'une autre origine que la première membrane (2), la première et la seconde membranes étant réticulées photochimiquement.

7. Membrane composite selon la revendication 6 **caractérisée en ce qu'**elle comprend deux premières membranes (2) et une seconde membrane (3), la seconde membrane (3) étant disposée entre les deux premières membranes (2).

8. Membrane composite selon la revendication 6 ou la revendication 7 caractérisée en ce les premières et secondes membres (2, 3) de la membrane composite (1) sont comprimées l'une contre l'autre et réticulées l'une avec l'autre.

9. Procédé de préparation d'un produit de construction tissulaire transplantable selon l'une quelconque des revendications 1 à 5 comprenant
(a) la préparation d'une membrane composite (1) comprenant au moins une première membrane biocompatible contenant du collagène (2) et une seconde membrane biocompatible contenant du collagène (3), la première membrane (2) et la seconde membrane (3) étant adjacentes l'une à l'autre par leurs faces planes, la première membrane (2) étant d'origine équine ou bovine et la seconde membrane (3) étant d'origine animale ou humaine, la seconde membrane (3) étant d'une autre origine que la première membrane; sous réticulation photochimique de la première membrane et de la deuxième membrane et,
(b) la formation d'une ou plusieurs couches (4) de cellules de tissu muqueux autologues orales sur l'une des deux ou sur les deux faces planes externes de la membrane composite (1).

10. Procédé selon la revendication 9, **caractérisé en ce que** l'étape (a) comprend les étapes consistant à
(a1) fournir deux premières membranes (2) et convertir les premières membranes (2) dans un état gonflés ;
(a2) fournir une seconde membrane (3) et lyophiliser la seconde membrane (3) ;
(a3) disposer la seconde membrane (3) entre les deux premières membranes (2) ; et
(a4) préparer un composite (1) des premières membranes (2) et de la seconde membrane (3).

11. Procédé selon la revendication 9 ou la revendication 10, **caractérisé en ce que** la membrane composite (1) est préparée par compression et par réticulation des premières et secondes membranes (2, 3).

12. Procédé selon l'une quelconque des revendications 9 à 11, **caractérisé en ce que** les cellules de tissu muqueux autologues orales sont appliquées sur l'une des deux ou sur les deux faces planes externes de la membrane composite (1) et qu'elles sont mises en culture de manière à former une ou plusieurs couches (4).
